(19) Europäisches Patentamt
European Patent Office
Office européen des brevets

(11) **EP 4 413 918 A1**

(12) **EUROPEAN PATENT APPLICATION**
published in accordance with Art. 153(4) EPC

(43) Date of publication:
**14.08.2024 Bulletin 2024/33**

(21) Application number: **21959833.1**

(22) Date of filing: **04.10.2021**

(51) International Patent Classification (IPC):
**A61B 5/0245** (2006.01)  **A61B 5/11** (2006.01)

(52) Cooperative Patent Classification (CPC):
**A61B 5/0245; A61B 5/11**

(86) International application number:
**PCT/JP2021/036642**

(87) International publication number:
**WO 2023/058090 (13.04.2023 Gazette 2023/15)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Nisshinbo Singapore Pte. Ltd.**
**Singapore Land Tower, 048623 (SG)**

(72) Inventors:
• **TSUSHIMA, Kengo**
**Singapore Pte. Ltd., 50 Raffles Place, 08-06**
**Singapore Land Tower, 048623 (SG)**

• **SAITO, Kazuo**
**Singapore Pte. Ltd., 50 Raffles Place, 08-06**
**Singapore Land Tower, 048623 (SG)**
• **FUJII, Minori**
**Singapore Pte. Ltd., 50 Raffles Place, 08-06**
**Singapore Land Tower, 048623 (SG)**

(74) Representative: **v. Bezold & Partner Patentanwälte - PartG mbB**
**Ridlerstraße 57**
**80339 München (DE)**

(54) **HEARTBEAT/RESPIRATION OUTPUT DEVICE AND HEARTBEAT/RESPIRATION OUTPUT PROGRAM**

(57)     This disclosure is a heartbeat/respiration output device M that includes a heartbeat/respiration extraction unit 1 that extracts a frequency component caused by a micro vibration of heartbeat and/or respiration from a radar signal or an ultrasound signal reflecting off a body surface of a patient P, a temporal change calculation unit 2 that calculates an amplitude temporal change or a phase temporal change of the frequency component caused by a micro vibration of heartbeat and/or respiration, a sound signal modulation unit 4 that performs amplitude modulation or frequency modulation on a sound signal having an audible band frequency based on the amplitude temporal change or the phase temporal change of the frequency component caused by a micro vibration of heartbeat and/or respiration, and a heartbeat/respiration output unit 5 that outputs the sound signal after amplitude modulation or frequency modulation.

FIG. 1

EP 4 413 918 A1

**Description**

TECHNICAL FIELD

This disclosure relates to a technique of listening to heartbeat and/or respiration like a stethoscope while enabling the alleviation of burdens on doctors or nurses, infection risk reduction, remote medical examination and treatment, early disease detection, and future predictive research based on radar signals or ultrasound signals reflecting off a body surface.

BACKGROUND ART

Patent Literature 1 and the like disclose a technique of calculating a heart rate and a respiration rate while enabling the alleviation of burdens on doctors or nurses, infection risk reduction, remote medical examination and treatment, early disease detection, and future predictive research based on radar signals reflecting off a body surface.

CITATION LIST

Patent Literature

Patent Literature 1: JP-A-2019-129996

SUMMARY OF INVENTION

Technical Problem

However, according to Patent Literature 1 and the like, if an irradiation direction of the radar signals is not accurately adjusted, the heart rate and the respiration rate may be incorrectly calculated. According to Patent Literature 1 and the like, the truth or falsehood of alerts was not correctly determined even if the heart rate and the respiration rate were incorrectly calculated.

Therefore, in order to solve the above-described problem, an object of this disclosure is to, based on a radar signal (including an ultrasound signal) reflecting off a body surface, when calculating a heart rate and/or a respiration rate, accurately adjust an irradiation direction of the radar signal or the ultrasound signal and correctly determine the truth or falsehood of alerts of the heart rate and/or the respiration rate, while enabling the alleviation of burdens on doctors or nurses, infection risk reduction, remote medical examination and treatment, early disease detection, and future predictive research.

Solution to Problem

In order to solve the above-described problem, an amplitude temporal change or a phase temporal change of a frequency component caused by a micro vibration of heartbeat and/or respiration is calculated from a radar signal or an ultrasound signal reflecting off a body surface. Then, amplitude modulation or frequency modulation is performed on a sound signal having an audible band frequency based on the amplitude temporal change or the phase temporal change of the frequency component caused by a micro vibration of heartbeat and/or respiration. Doctors or nurses can then listen to the heartbeat and/or the respiration like a stethoscope.

Specifically, this disclosure is a heartbeat/respiration output device including: a heartbeat/respiration extraction unit that extracts a frequency component caused by a micro vibration of heartbeat and/or respiration from a radar signal or an ultrasound signal reflecting off a body surface and calculates an amplitude temporal change or a phase temporal change of the frequency component caused by a micro vibration of heartbeat and/or respiration; and a heartbeat/respiration output unit that performs amplitude modulation or frequency modulation on a sound signal having an audible band frequency based on the amplitude temporal change or the phase temporal change of the frequency component caused by a micro vibration of heartbeat and/or respiration and outputs the sound signal after amplitude modulation or frequency modulation.

This configuration (1) allows accurately adjusting an irradiation direction of the radar signal or the ultrasound signal so that the doctors or the nurses can experimentally listen to the heartbeat and/or the respiration, (2) allows the doctors or the nurses to correctly determine the truth or falsehood of alerts of a heart rate and/or a respiration rate by directly listening to the heartbeat and/or the respiration, and (3) is applicable to the alleviation of burdens on the doctors or the nurses, infection risk reduction, remote medical examination and treatment, early disease detection, and future predictive research.

This disclosure is the heartbeat/respiration output device in which the heartbeat/respiration output unit performs one modulation of amplitude modulation and frequency modulation on the sound signal based on the amplitude temporal change or the phase temporal change of the frequency component caused by a micro vibration of heartbeat and performs another modulation of amplitude modulation and frequency modulation on the sound signal based on the amplitude temporal change or the phase temporal change of the frequency component caused by a micro vibration of respiration.

This configuration allows listening to the heartbeat and the respiration as a magnitude and a pitch of the sound signal, respectively, or listening to the heartbeat and the respiration as a pitch and a magnitude of the sound signal, respectively, resulting in allowing listening to the heartbeat and the respiration simultaneously and separately.

This disclosure is the heartbeat/respiration output device in which the heartbeat/respiration extraction unit calculates the phase temporal change of the frequency component caused by a micro vibration of respiration, with the phase temporal change of the frequency component caused by a micro vibration of heartbeat removed by extracting and then performing complex multiplication of positive and negative frequency components caused by a micro vibration of heartbeat.

With this configuration, since the frequency component (on the order of $\pm 10$ or $10^2$ Hz) caused by the micro vibration of the heartbeat is extracted when listening to the respiration, without extracting the frequency component (DC component including an extremely low frequency component) caused by the micro vibration of the respiration, it is possible to ensure the improvement of robustness and the reduction of the effects of disturbances, and it is only necessary to consider a reflected signal from a chest without considering a reflected signal from an abdomen. Note that disturbances, such as louvers of air conditioners, curtains swayed by the wind and the movements of nurses, are mainly included in the DC component and are close to the frequency component caused by the micro vibration of the respiration, therefore making it difficult to improve the robustness and reduce the effects of the disturbances. In addition, if the reflected signal from the abdomen and the reflected signal from the chest are synthesized, the respiration rate may be falsely recognized by a factor of two depending on synthesis conditions.

This disclosure is the heartbeat/respiration output device in which the heartbeat/respiration extraction unit performs uprating on the amplitude temporal change or the phase temporal change having a low sampling frequency compared with the audible band frequency that the sound signal has, at a high uprating frequency compared with the audible band frequency that the sound signal has.

With this configuration, even when the amplitude temporal change or the phase temporal change of the frequency components caused by the micro vibrations of the heartbeat and/or the respiration is sampled at a "low sampling frequency," by performing uprating on the amplitude temporal change or the phase temporal change of the frequency components caused by the micro vibrations of the heartbeat and/or the respiration at a "high uprating frequency," the sound signal having the audible band frequency can be amplitude-modulated or frequency-modulated by modulated information after the uprating.

This disclosure is the heartbeat/respiration output device in which the heartbeat/respiration extraction unit performs any of low-pass filter processing after zero-padding processing on the amplitude temporal change or the phase temporal change, copy interpolation processing on the amplitude temporal change or the phase temporal change, and spline interpolation processing on the amplitude temporal change or the phase temporal change when performing uprating on the amplitude temporal change or the phase temporal change.

With this configuration, (1) using the low-pass filter processing after the zero-padding processing can improve accuracy on the uprating even though a calculation amount during the uprating increases, (2) using the copy interpolation processing can reduce the calculation amount during the uprating even though discontinuous noise on the uprating remains, and (3) using the spline interpolation processing can reduce the calculation amount during the uprating and improve the accuracy on the uprating.

This disclosure is a heartbeat/respiration output program for causing a computer to execute each processing step corresponding to each processing unit of the above-described heartbeat/respiration output device.

This configuration allows providing the program having the above-described effects.

Advantageous Effects of Invention

Thus, this disclosure allows, based on a radar signal (including an ultrasound signal) reflecting off a body surface, when calculating a heart rate and/or a respiration rate, accurately adjusting an irradiation direction of the radar signal or the ultrasound signal and correctly determining the truth or falsehood of alerts of the heart rate and/or the respiration rate, while enabling the alleviation of burdens on doctors or nurses, infection risk reduction, remote medical examination and treatment, early disease detection, and future predictive research.

BRIEF DESCRIPTION OF DRAWINGS

Fig. 1 is a diagram illustrating a configuration of a heartbeat/respiration output device of this disclosure.
Fig. 2 is a diagram illustrating a procedure of heartbeat/respiration output processing of this disclosure.
Fig. 3 is a drawing illustrating a specific example of heartbeat extraction processing of this disclosure.
Fig. 4 is a drawing illustrating a specific example of respiration extraction processing of this disclosure.
Fig. 5 is a drawing illustrating a specific example of heartbeat/respiration extraction processing of this disclosure.
Fig. 6 is a drawing illustrating a specific example of sound signal modulation processing of this disclosure.
Fig. 7 is a drawing illustrating a specific example of frequency uprating processing of this disclosure.
Fig. 8 is a drawing illustrating a specific example of the frequency uprating processing of this disclosure.
Fig. 9 is a drawing illustrating a specific example of the frequency uprating processing of this disclosure.
Fig. 10 is a drawing illustrating a specific example of the heartbeat/respiration output processing of this disclosure.
Fig. 11 is a drawing illustrating a specific example of the heartbeat/respiration output processing of this disclosure.

DESCRIPTION OF EMBODIMENTS

Embodiments of this disclosure will be described by referring to the accompanying drawings. The embodiments described below are examples of the implementation of this disclosure, and this disclosure is not limited to the following embodiments.

(Configuration of Heartbeat/Respiration Output Device of This Disclosure)

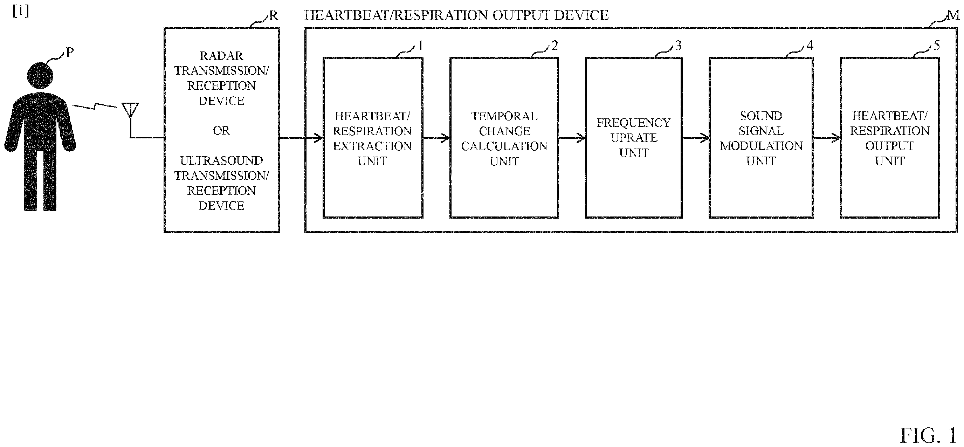

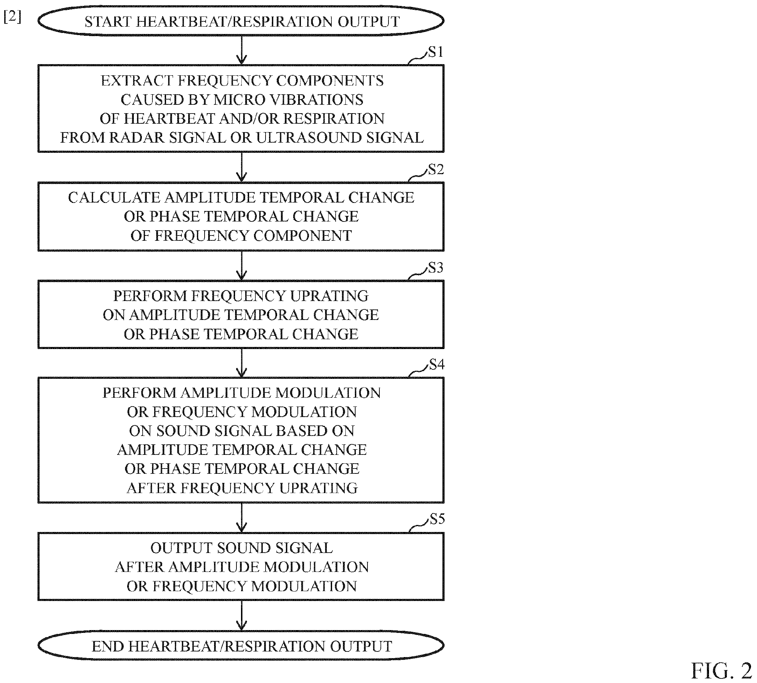

Fig. 1 illustrates a configuration of a heartbeat/respiration output device of this disclosure. Fig. 2 illustrates a procedure of heartbeat/respiration output processing of this disclosure. A heartbeat/respiration output device M is equipped with a heartbeat/respiration extraction unit 1, a temporal change calculation unit 2, a frequency uprate unit 3, a sound signal modulation unit 4, and a heartbeat/respiration output unit 5 and can be realized by installing a heartbeat/respiration output program illustrated in Fig. 2 on a computer.
A radar transmission/reception device R or an ultrasound transmission/reception device R transmits a radar signal or an ultrasound signal (carrier wave band) with which a body surface of a patient P is irradiated, receives the radar signal or the ultrasound signal (carrier wave band) reflecting off the body surface of the patient P, and converts the received radar signal or ultrasound signal to a baseband to output it. A radar method or an ultrasound method may be any of a CW method, an FMCW method, a standing wave method, and other methods. The radar signal or the ultrasound signal (carrier wave band) has a wavelength on the order of 1 mm to 10 mm, which is equal to on the order of a micro vibration width of the body surface of the patient P.
The heartbeat/respiration output device M calculates an amplitude temporal change or a phase temporal change of frequency components caused by micro vibrations of heartbeat and/or respiration from the radar signal or the ultrasound signal (baseband) reflecting off the body surface of the patient P. Then, based on the amplitude temporal change or the phase temporal change of the frequency components caused by the micro vibrations of the heartbeat and/or the respiration, amplitude modulation or frequency modulation is performed on a sound signal having an audible band frequency (on the order of 10 Hz to 10 kHz, especially on the order of 100 Hz). Doctors or nurses can then listen to the heartbeat and/or the respiration like a stethoscope.
Accordingly, an irradiation direction of the radar signal or the ultrasound signal can be accurately adjusted so that the doctors or the nurses can experimentally listen to the heartbeat and/or the respiration. Then, the doctors or the nurses can correctly determine the truth or falsehood of alerts of a heart rate and/or a respiration rate by directly listening to the heartbeat and/or the respiration. Furthermore, the heartbeat/respiration output device M is applicable to the alleviation of burdens on the doctors or the nurses, infection risk reduction, remote medical examination and treatment, early disease detection, and future predictive research. The following describes a specific example of each processing of the heartbeat/respiration output device M.

(Specific Example of Heartbeat/Respiration Extraction Processing of This Disclosure)

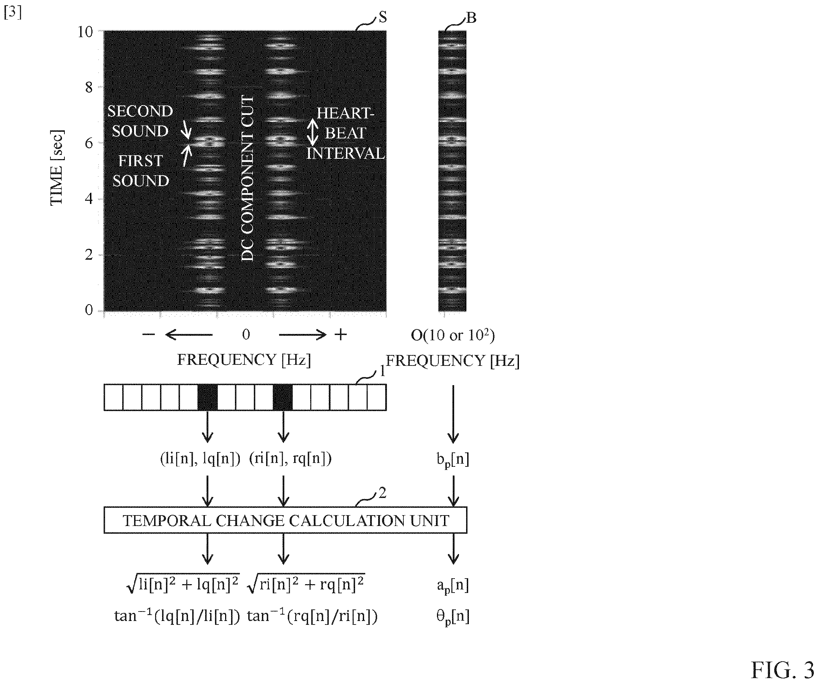

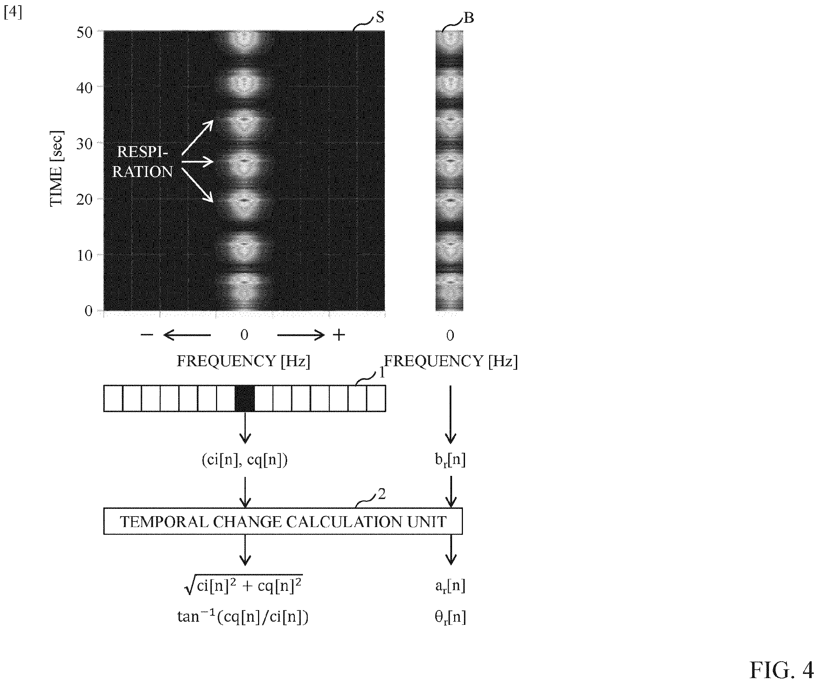

Figs. 3 and 4 illustrate specific examples of heartbeat/respiration extraction processing of this disclosure. The heartbeat/respiration extraction unit 1 extracts the frequency components (on the order of $\pm 10$ or $10^2$ Hz and/or a DC component including an extremely low frequency component) caused by the micro vibrations of the heartbeat and/or the respiration from the radar signal or the ultrasound signal (I/Q complex signal converted to the baseband) reflecting off the body surface of the patient P (Step S 1). Alternatively, the heartbeat/respiration extraction unit 1 extracts the frequency components (on the order of +10 or $10^2$ Hz and/or the DC component including an extremely low frequency component) caused by the micro vibrations of the heartbeat and/or the respiration from the radar signal or the ultrasound signal (real number signal converted to the baseband) reflecting off the body surface of the patient P (Step S 1).
In Fig. 3, the heartbeat/respiration extraction unit 1 calculates a spectrogram S that shows the temporal change of each frequency component from the radar signal or the ultrasound signal (I/Q complex signal converted to the baseband) reflecting off the body surface of the patient P. Then, from the spectrogram S, the frequency component (on the order of $\pm 10$ or $10^2$ Hz) caused by the micro vibration of the heartbeat is extracted. Alternatively, the heartbeat/respiration extraction unit 1 calculates a band-pass filter result B in the frequency component caused by the micro vibration of the heartbeat from the radar signal or the ultrasound signal (real number signal converted to the baseband) reflecting off the body surface of the patient P. Then, in both cases, about once or twice in 2 seconds, small amplitude peaks caused by the micro vibration of the heartbeat are extracted, and close amplitude peaks of a first sound and a second sound in one heartbeat are extracted.

Here, the heartbeat/respiration extraction unit 1 extracts (ri[n], rq[n]) (r is a positive frequency, *i* and *q* are *i* and *q* components, respectively, and *n* is time) as a positive frequency component caused by the micro vibration of the heartbeat and (li[n], lq[n]) (*l* is a negative frequency, *i* and *q* are *i* and *q* components, respectively, and *n* is time) as a negative frequency component caused by the micro vibration of the heartbeat from the radar signal or the ultrasound signal (I/Q complex signal converted to the baseband) reflecting off the body surface of the patient **P**. Alternatively, the heartbeat/respiration extraction unit 1 extracts $b_p$[n] (**b** is a pass frequency band of the band-pass filter result *B, p* is heartbeat, and *n* is time) as a frequency component caused by the micro vibration of the heartbeat from the radar signal or the ultrasound signal (real number signal converted to the baseband) reflecting off the body surface of the patient P.

In Fig. 4, the heartbeat/respiration extraction unit 1 calculates the spectrogram S that shows the temporal change of each frequency component from the radar signal or the ultrasound signal (I/Q complex signal converted to the baseband) reflecting off the body surface of the patient P. Then, from the spectrogram S, the frequency component (DC component including an extremely low frequency component) caused by the micro vibration of the respiration is extracted. Alternatively, the heartbeat/respiration extraction unit 1 calculates the band-pass filter result B in the frequency component caused by the micro vibration of the respiration from the radar signal or the ultrasound signal (real number signal converted to the baseband) reflecting off the body surface of the patient P. Then, in both cases, about once or twice in 10 seconds, amplitude peaks caused by the micro vibration of the respiration are extracted, and about three times between the respiration amplitude peaks, amplitude peaks caused by the micro vibration of the heartbeat are also extracted.

Here, the heartbeat/respiration extraction unit 1 extracts (ci[n], cq[n]) (c is a center frequency, *i* and *q* are *i* and *q* components, respectively, and *n* is time) as a frequency component caused by the micro vibration of the respiration from the radar signal or the ultrasound signal (I/Q complex signal converted to the baseband) reflecting off the body surface of the patient P. Alternatively, the heartbeat/respiration extraction unit 1 extracts $b_r$[n] (**b** is a pass frequency band of the band-pass filter result B, *r* is respiration, and *n* is time) as a frequency component caused by the micro vibration of the respiration from the radar signal or the ultrasound signal (real number signal converted to the baseband) reflecting off the body surface of the patient P.

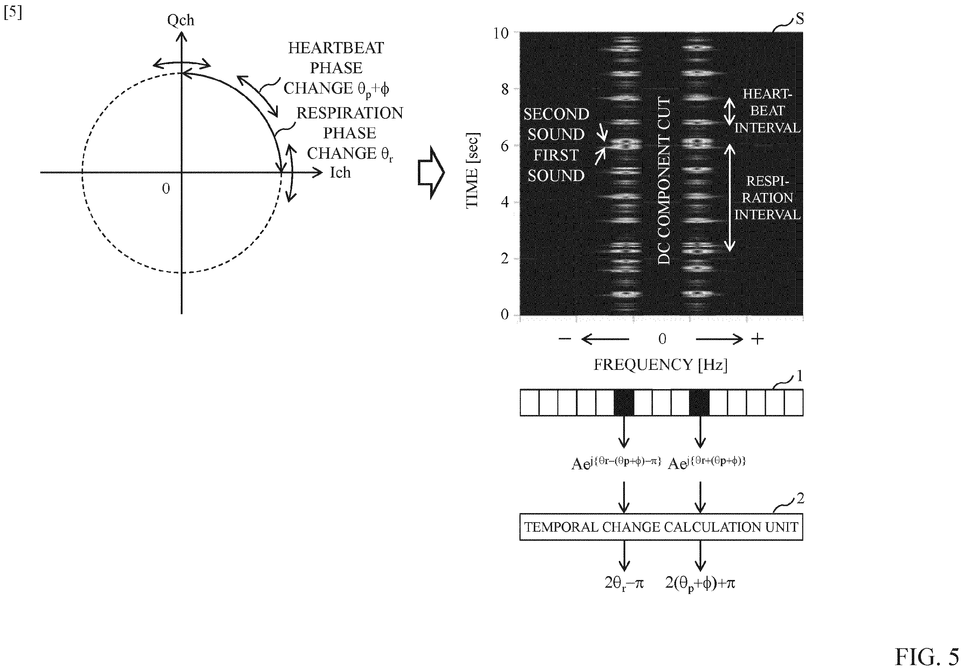

Fig. 5 also illustrates a specific example of the heartbeat/respiration extraction processing of this disclosure. The heartbeat/respiration extraction unit 1 extracts the positive and negative frequency components (on the order of $\pm 10$ or $10^2$ Hz) caused by the micro vibration of the heartbeat from the radar signal or the ultrasound signal (I/Q complex signal converted to the baseband) reflecting off the body surface of the patient P (Step S1). Then, the heartbeat/respiration extraction unit 1 performs complex multiplication/division of the positive and negative frequency components (on the order of $\pm 10$ or $10^2$ Hz) caused by the micro vibration of the heartbeat (Step S1).

The left section of Fig. 5 describes a principle of complex multiplication/division processing of the heartbeat/respiration extraction unit 1. On the radar signal or the ultrasound signal (carrier wave band) reflecting off the body surface of the patient P, heartbeat phase modulation caused by the micro vibration of the heartbeat is performed, and respiration phase modulation caused by the micro vibration of the respiration is also performed. Here, on the radar signal or the ultrasound signal (carrier wave band) reflecting off the body surface of the patient P, although heartbeat amplitude modulation is also performed, the heartbeat amplitude modulation is uncertain compared with the heartbeat phase modulation and therefore disadvantageous for highly accurate heartbeat extraction, and although respiration amplitude modulation is also performed, the respiration amplitude modulation is uncertain compared with the respiration phase modulation and therefore disadvantageous for highly accurate respiration extraction.

In the right section of Fig. 5, the heartbeat/respiration extraction unit 1 extracts $Ae^{j\{\theta r+(\theta p+\phi)\}}$ (A is an amplitude, $\theta_r$ is a respiration phase change, $\theta_p$ is a heartbeat phase change, and $\phi$ is an initial phase of the heartbeat phase change) as a positive frequency component caused by the micro vibration of the heartbeat and extracts $Ae^{j\{\theta r-(\theta p+\phi)-\pi\}}$ ($\pi$ is a phase difference between upper and lower sideband waves of phase modulation) as a negative frequency component caused by the micro vibration of the heartbeat from the radar signal or the ultrasound signal (I/Q complex signal converted to the baseband) reflecting off the body surface of the patient P.

Then, the heartbeat/respiration extraction unit 1 calculates $Ae^{j\{\theta r+(\theta p+\phi)\}} * Ae^{j\{\theta r-(\theta p+\phi)-\pi\}} = |A|^2 e^{j(2\theta r-\pi)}$ as complex multiplication of the positive and negative frequency components. Accordingly, from complex multiplication of the positive and negative frequency components $|A|^2 e^{j(2\theta r-\pi)}$, the respiration phase change $\theta_r$ caused by the micro vibration of the respiration, with the heartbeat phase change $\theta_p+\phi$ caused by the micro vibration of the heartbeat removed, can be extracted.

Thus, since the frequency component (on the order of $\pm 10$ or $10^2$ Hz) caused by the micro vibration of the heartbeat is extracted when listening to the respiration, without extracting the frequency component (DC component including an extremely low frequency component) caused by the micro vibration of the respiration, it is possible to ensure the improvement of robustness and the reduction of the effects of disturbances, and it is only necessary to consider a reflected signal from a chest without considering a reflected signal from an abdomen. Note that disturbances, such as louvers of air conditioners, curtains swayed by the wind and the movements of nurses, are mainly included in the DC component and are close to the frequency component caused by the micro vibration of the respiration, therefore making it difficult to improve the robustness and reduce the effects of the disturbances. In addition, if the reflected signal from the abdomen and the reflected signal from the chest are synthesized, the respiration rate may be falsely recognized by a factor of two depending on synthesis conditions.

Meanwhile, the heartbeat/respiration extraction unit 1 calculates $Ae^{j\{\theta r+(\theta p+\phi)\}}/Ae^{j\{(\theta r-(\theta p+\phi)-\pi\}} = e^{j\{2(\theta p+\phi)+\pi\}}$ as complex division of the positive and negative frequency components. Accordingly, from complex division of the positive and negative frequency components $e^{j\{2(\theta p+\phi)+\pi\}}$, the heartbeat phase change $\theta_\rho+\phi$ caused by the micro vibration of the heartbeat, with the respiration phase change $\theta_r$ caused by the micro vibration of the respiration removed, can be extracted.

Thus, since the frequency component (on the order of $\pm 10$ or $10^2$ Hz) caused by the micro vibration of the heartbeat is extracted when listening to the heartbeat, it is possible to ensure the improvement of robustness and the reduction of the effects of disturbances, but it should be noted that in one heartbeat, whether the first sound and the second sound are in-phase state, inverse phase state, or other states vary depending on individual differences or animal species.

(Specific Example of Sound Signal Modulation Processing of This Disclosure)

Prior to sound signal modulation processing of this disclosure, the temporal change calculation unit 2 calculates the amplitude temporal change or the phase temporal change of the frequency components (on the order of $\pm 10$ or $10^2$ Hz and/or the DC component including an extremely low frequency component) caused by the micro vibrations of the heartbeat and/or the respiration (Step S2).

In Fig. 3, the temporal change calculation unit 2 calculates $\sqrt{(ri[n]^2+rq[n]^2)}$, $\sqrt{(li[n]^2\pm lq[n]^2)}$, or $a_p[n]$ (= amplitude of $b_p[n]$) as the amplitude temporal change of the frequency component (on the order of $\pm 10$ or $10^2$ Hz) caused by the micro vibration of the heartbeat. Alternatively, the temporal change calculation unit 2 calculates $\tan^{-1}(rq[n]/ri[n])$, $\tan^{-1}(lq[n]/li[n])$, or $\theta_p[n]$ (= phase of $b_p[n]$) as the phase temporal change of the frequency component (on the order of $\pm 10$ or $10^2$ Hz) caused by the micro vibration of the heartbeat.

In Fig. 4, the temporal change calculation unit 2 calculates $\sqrt{(ci[n]^2+cq[n]^2)}$ or $a_r[n]$ (= amplitude of $b_r[n]$) as the amplitude temporal change of the frequency component (DC component including an extremely low frequency component) caused by the micro vibration of the respiration. Alternatively, the temporal change calculation unit 2 calculates $\tan^{-1}(cq[n]/ci[n])$ or $\theta_r[n]$ (= phase of $b_r[n]$) as the phase temporal change of the frequency component (DC component including an extremely low frequency component) caused by the micro vibration of the respiration.

In Fig. 5, the temporal change calculation unit 2 calculates $2\theta_r-\pi$ (extracted argument of e) as the phase temporal change of the frequency component (DC component including an extremely low frequency component) caused by the micro vibration of the respiration. Alternatively, the temporal change calculation unit 2 calculates $2(\theta_\rho+\phi)+\pi$ (extracted argument of e) as the phase temporal change of the frequency component (on the order of $\pm 10$ or $10^2$ Hz) caused by the micro vibration of the heartbeat.

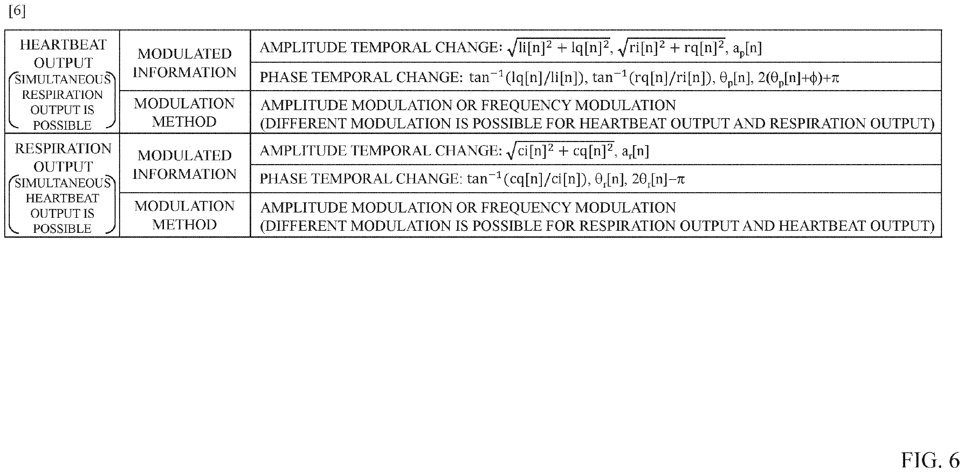

Fig. 6 illustrate a specific example of the sound signal modulation processing of this disclosure. The sound signal modulation unit 4 performs amplitude modulation or frequency modulation on a sound signal having an audible band frequency based on the amplitude temporal change or the phase temporal change of the frequency components caused by the micro vibrations of the heartbeat and/or the respiration (Step S4). The heartbeat/respiration output unit 5 outputs the sound signal after amplitude modulation or frequency modulation (Step S5).

In the upper stage of Fig. 6, at heartbeat output, modulated information is the amplitude temporal change ($\sqrt{(ri[n]^2+rq[n]^2)}$, $\sqrt{(li[n]^2+lq[n]^2)}$, $a_p[n]$) or the phase temporal change ($\tan^{-1}(rq[n]/ri[n])$, $\tan^{-1}(lq[n]/li[n])$, $\theta_p[n]$, $2(\theta_p[n]+\phi)+\pi$), and the modulation method is amplitude modulation or frequency modulation.

In the lower stage of Fig. 6, at respiration output, modulated information is the amplitude temporal change ($\sqrt{(ci[n]^2+cq[n]^2)}$, $a_r[n]$) or the phase temporal change ($\tan^{-1}(cq[n]/ci[n])$, $\theta_r[n]$, $2\theta_r[n]-\pi$), and the modulation method is amplitude modulation or frequency modulation.

The sound signal modulation unit 4 may perform "one" modulation of amplitude modulation and frequency modulation on the sound signal based on the amplitude temporal change or the phase temporal change of the frequency component caused by the micro vibration of the "heartbeat" and may perform "the other" modulation of amplitude modulation and frequency modulation on the sound signal based on the amplitude temporal change or the phase temporal change of the frequency component caused by the micro vibration of the "respiration" (Step S4).

For example, at the heartbeat output, the modulated information is assumed to be √(ri[n]²+rq[n]²), and at the respiration output, the modulated information is assumed to be $2\theta_r[n]-\pi$. Then, when amplitude modulation is performed at the heartbeat output and frequency modulation is performed at the respiration output, the sound signal after modulation is expressed by the first equation of Math. 1. When frequency modulation is performed at the heartbeat output and amplitude modulation is performed at the respiration output, the sound signal after modulation is expressed by the second equation of Math. 1. Here, S[n] is a sound signal after **modulation, *f* is** an audible band frequency, and $\beta$ is a modulation index.

[Math. 1]

$$S[n] = \sqrt{ri[n]^2 + rq[n]^2}\cos\left[2\pi fn + \beta\sum_{k=0}^{n}(2\theta_r[k] - \pi)\right]S[n] = (2\theta_r[n] - \pi)$$

$$\cos\left[2\pi fn + \beta\sum_{k=0}^{n}\sqrt{ri[k]^2 + rq[k]^2}\right][0047]$$

Thus, the heartbeat and the respiration can be listened to as a magnitude and a pitch of the sound signal, respectively, or the heartbeat and the respiration can be listened to as a pitch and a magnitude of the sound signal, respectively, resulting in allowing listening to the heartbeat and the respiration simultaneously and separately.

The sound signal modulation unit 4 may perform "one" modulation of amplitude modulation and frequency modulation on the sound signal based on the amplitude temporal change or the phase temporal change of the frequency component caused by the micro vibration of the "heartbeat" and may perform "the same" modulation of amplitude modulation and frequency modulation on the sound signal based on the amplitude temporal change or the phase temporal change of the frequency component caused by the micro vibration of the "respiration" (Step S4).

For example, at the heartbeat output, the modulated information is assumed to be √(ri[n]²+rq[n]²), and at the respiration output, the modulated information is assumed to be $2\theta_r[n]-\pi$. Then, when frequency modulation is performed at the heartbeat output and frequency modulation is performed at the respiration output, the sound signal after modulation is expressed by the first equation of Math. 2. When amplitude modulation is performed at the heartbeat output and amplitude modulation is performed at the respiration output, the sound signal after modulation is expressed by the second equation of Math. 2. Here, S[n] is a sound signal after modulation, *f* is an audible band frequency, and $\beta$ is a modulation index.

[Math. 2]

$$S[n] = \cos\left[2\pi fn + \beta\sum_{k=0}^{n}\{\sqrt{ri[k]^2 + rq[k]^2} + (2\theta_r[k] - \pi)\}\right]$$

$$S[n] = \{\sqrt{ri[n]^2 + rq[n]^2} + (2\theta_r[n] - \pi)\}\cos[2\pi fn]$$

Thus, both the heartbeat and the respiration can be listened to as the pitch of the sound signal, or both the heartbeat and the respiration can be listened to as the magnitude of the sound signal, resulting in allowing listening to the heartbeat and the respiration simultaneously and separately, although this case is inferior compared with the case of Math. 1.

(Specific Example of Frequency Uprating Processing of This Disclosure)

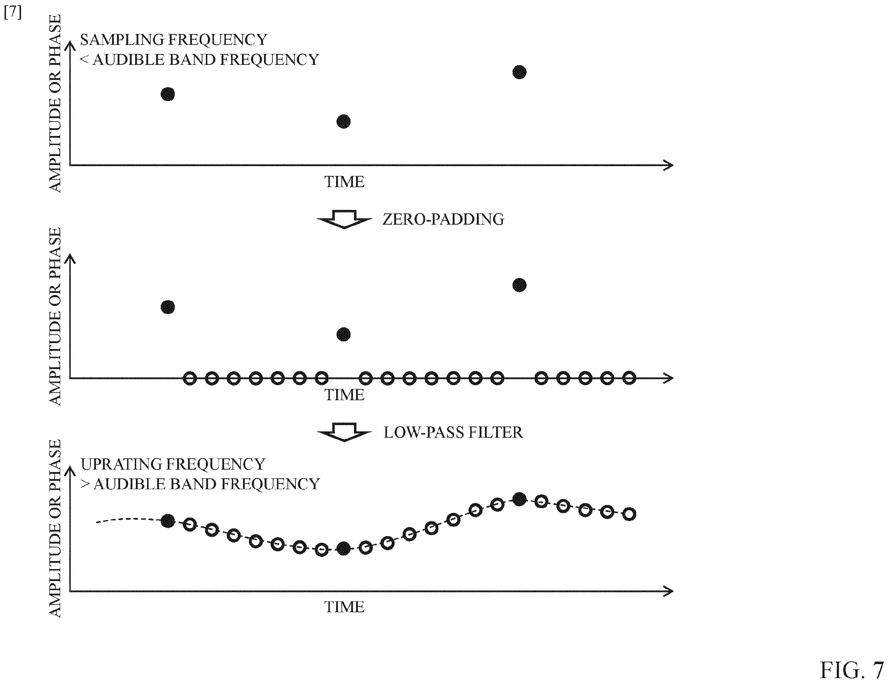

Figs. 7 to 9 illustrate specific examples of frequency uprating processing of this disclosure. The frequency uprate unit 3 performs uprating on the amplitude temporal change or the phase temporal change calculated in Step S2, which has a low sampling frequency compared with the audible band frequency that the sound signal has, at a high uprating frequency compared with the audio band frequency that the sound signal has (Step S3).

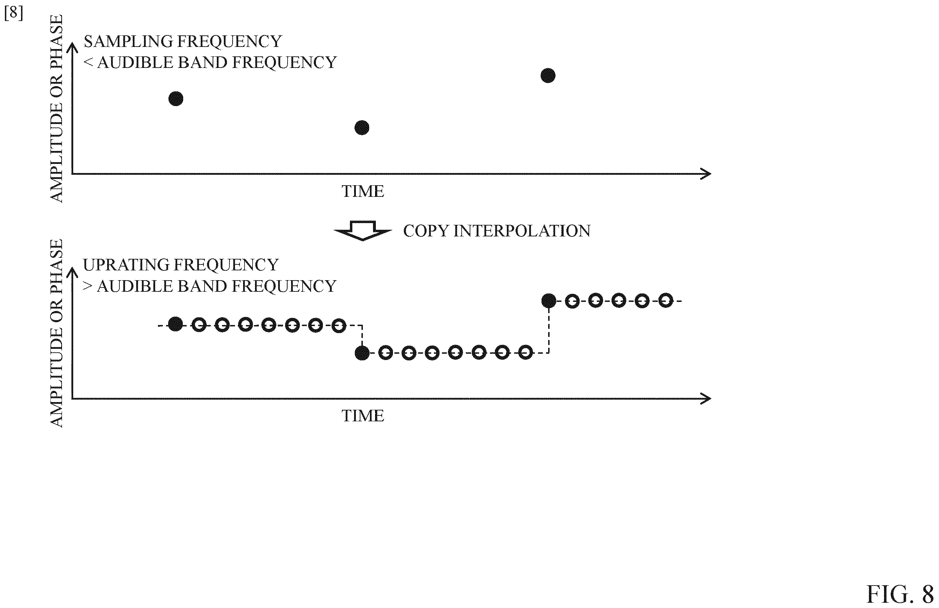

In Fig. 7, the frequency uprate unit 3 performs low-pass filter processing after zero-padding processing on the amplitude temporal change or the phase temporal change when performing uprating on the amplitude temporal change or the phase temporal change. Then, even though a calculation amount during the uprating increases, accuracy on the uprating can be improved (there is no discontinuous noise of Fig. 8).

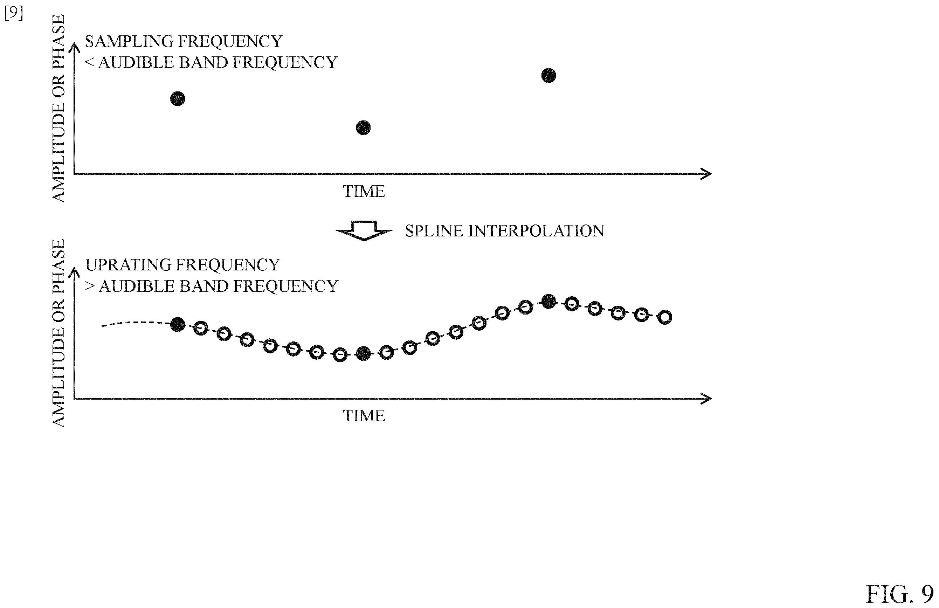

In Fig. 8, the frequency uprate unit 3 performs copy interpolation processing on the amplitude temporal change or the phase temporal change when performing uprating on the amplitude temporal change or the phase temporal change (without performing the zero-padding processing). Then, even though the discontinuous noise on the uprating remains (there is no continuity of Figs. 7 and 9), the calculation amount during the uprating can be reduced.

In Fig. 9, the frequency uprate unit 3 performs spline interpolation processing on the amplitude temporal change or the phase temporal change when performing uprating on the amplitude temporal change or the phase temporal change (without performing the zero-padding processing). Then, the calculation amount during the uprating can be reduced, and the accuracy on the uprating can be improved (there is no discontinuous noise of Fig. 8).

Accordingly, even when the amplitude temporal change or the phase temporal change of the frequency components caused by the micro vibrations of the heartbeat and/or the respiration is sampled at a "low sampling frequency," by performing uprating on the amplitude temporal change or the phase temporal change of the frequency components caused by the micro vibrations of the heartbeat and/or the respiration at a "high uprating frequency," the sound signal having the audible band frequency can be amplitude-modulated or frequency-modulated by modulated information after the uprating.

(Specific Example of Heartbeat/Respiration Output Processing of This Disclosure)

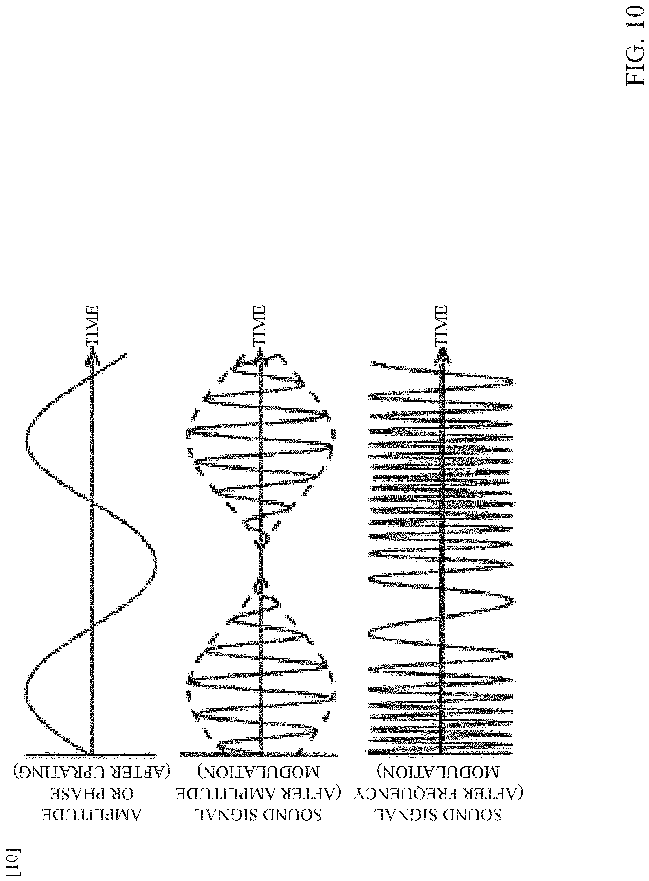

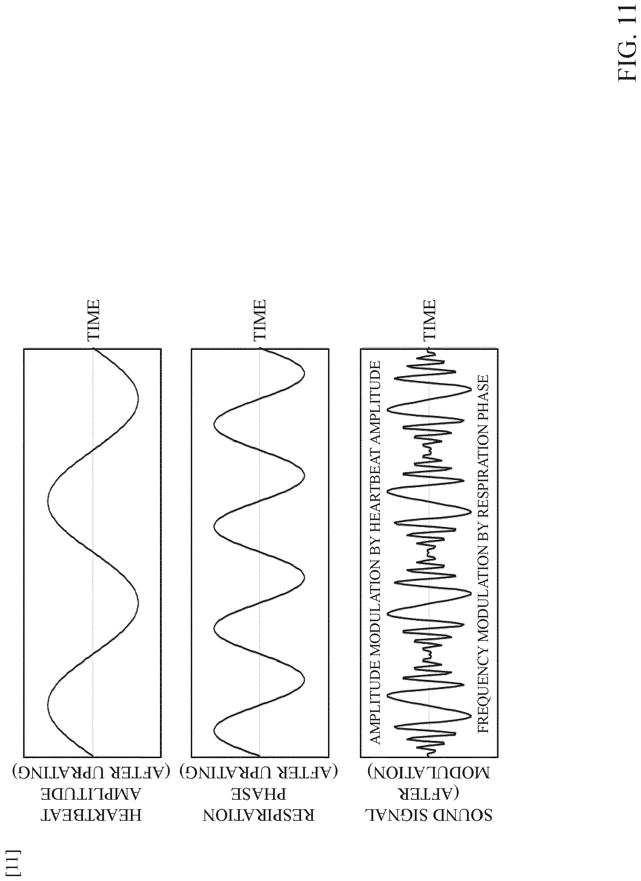

Figs. 10 and 11 illustrate specific examples of the heartbeat/respiration output processing of this disclosure. The sound signal modulation unit 4 performs amplitude modulation or frequency modulation on a sound signal having an audible band frequency based on the amplitude temporal change or the phase temporal change on which frequency uprating is performed in Step S3 (Step S4). The heartbeat/respiration output unit 5 outputs the sound signal that is amplitude-modulated or is frequency-modulated in Step S4 (Step S5). In Figs. 10 and 11, output objects, modulated information, and modulation methods are different.

The upper stage of Fig. 10 illustrates the amplitude temporal change or the phase temporal change of the heartbeat or the respiration on which frequency uprating is performed in Step S3, and the amplitude temporal change or the phase temporal change is outside the audible band frequency but serves as modulated information. The middle stage of Fig. 10 illustrates the sound signal that is amplitude-modulated in Step S4, and this sound signal is within the audible band frequency. The lower stage of Fig. 10 illustrates the sound signal that is frequency-modulated in Step S4, and this sound signal is also within the audible band frequency.

The upper stage of Fig. 11 illustrates the amplitude temporal change of the heartbeat on which frequency uprating is performed in Step S3, and this amplitude temporal change is outside the audible band frequency but serves as modulated information. The middle stage of Fig. 11 illustrates the phase temporal change of the respiration on which frequency uprating is performed in Step S3, and this phase temporal change is outside the audible band frequency but serves as modulated information. The lower stage of Fig. 11 illustrates the sound signal that is amplitude-modulated by the amplitude temporal change of the heartbeat and is frequency-modulated by the phase temporal change of the respiration in Step S4, and this sound signal is within the audible band frequency.

INDUSTRIAL APPLICABILITY

The heartbeat/respiration output device and the heartbeat/respiration output program of this disclosure allow, based on a radar signal (including an ultrasound signal) reflecting off a body surface, when calculating a heart rate and/or a respiration rate, accurately adjusting a irradiation direction of the radar signal or the ultrasound signal and correctly determining the truth or falsehood of alerts of the heart rate and/or the respiration rate, while enabling the alleviation of burdens on doctors or nurses, infection risk reduction, remote medical examination and treatment, early disease detection, and future predictive research.

REFERENCE SIGNS LIST

P     Patient
R     Radar transmission/reception device, ultrasound transmission/reception device
M     Heartbeat/respiration output device
S     Spectrogram
B     Band-pass filter result
1     Heartbeat/respiration extraction unit
2     Temporal change calculation unit
3     Frequency uprate unit
4     Sound signal modulation unit

5    Heartbeat/respiration output unit

**Claims**

1. A heartbeat/respiration output device comprising:

   a heartbeat/respiration extraction unit that extracts a frequency component caused by a micro vibration of heartbeat and/or respiration from a radar signal or an ultrasound signal reflecting off a body surface and calculates an amplitude temporal change or a phase temporal change of the frequency component caused by a micro vibration of heartbeat and/or respiration; and
   a heartbeat/respiration output unit that performs amplitude modulation or frequency modulation on a sound signal having an audible band frequency based on the amplitude temporal change or the phase temporal change of the frequency component caused by a micro vibration of heartbeat and/or respiration and outputs the sound signal after amplitude modulation or frequency modulation.

2. The heartbeat/respiration output device according to claim 1, wherein
   the heartbeat/respiration output unit performs one modulation of amplitude modulation and frequency modulation on the sound signal based on the amplitude temporal change or the phase temporal change of the frequency component caused by a micro vibration of heartbeat and performs another modulation of amplitude modulation and frequency modulation on the sound signal based on the amplitude temporal change or the phase temporal change of the frequency component caused by a micro vibration of respiration.

3. The heartbeat/respiration output device according to claim 1 or 2, wherein
   the heartbeat/respiration extraction unit calculates the phase temporal change of the frequency component caused by a micro vibration of respiration, with the phase temporal change of the frequency component caused by a micro vibration of heartbeat removed by extracting and then performing complex multiplication of positive and negative frequency components caused by a micro vibration of heartbeat.

4. The heartbeat/respiration output device according to any one of claims 1 to 3, wherein
   the heartbeat/respiration extraction unit performs uprating on the amplitude temporal change or the phase temporal change having a low sampling frequency compared with the audible band frequency that the sound signal has, at a high uprating frequency compared with the audible band frequency that the sound signal has.

5. The heartbeat/respiration output device according to claim 4, wherein
   the heartbeat/respiration extraction unit performs any of low-pass filter processing after zero-padding processing on the amplitude temporal change or the phase temporal change, copy interpolation processing on the amplitude temporal change or the phase temporal change, and spline interpolation processing on the amplitude temporal change or the phase temporal change when performing uprating on the amplitude temporal change or the phase temporal change.

6. A heartbeat/respiration output program for causing a computer to execute each processing step corresponding to each processing unit of the heartbeat/respiration output device according to any one of claims 1 to 5.

[1]

P

R    HEARTBEAT/RESPIRATION OUTPUT DEVICE    M

| RADAR TRANSMISSION/ RECEPTION DEVICE OR ULTRASOUND TRANSMISSION/ RECEPTION DEVICE | HEARTBEAT/ RESPIRATION EXTRACTION UNIT | TEMPORAL CHANGE CALCULATION UNIT | FREQUENCY UPRATE UNIT | SOUND SIGNAL MODULATION UNIT | HEARTBEAT/ RESPIRATION OUTPUT UNIT |

1   2   3   4   5

10

FIG. 1

[2]

```
┌─────────────────────────────────────────┐
│      START HEARTBEAT/RESPIRATION OUTPUT  │
└─────────────────────────────────────────┘
                    │
                    ▼                    S1
┌─────────────────────────────────────────┐
│        EXTRACT FREQUENCY COMPONENTS      │
│         CAUSED BY MICRO VIBRATIONS       │
│      OF HEARTBEAT AND/OR RESPIRATION     │
│  FROM RADAR SIGNAL OR ULTRASOUND SIGNAL  │
└─────────────────────────────────────────┘
                    │
                    ▼                    S2
┌─────────────────────────────────────────┐
│    CALCULATE AMPLITUDE TEMPORAL CHANGE   │
│         OR PHASE TEMPORAL CHANGE         │
│          OF FREQUENCY COMPONENT          │
└─────────────────────────────────────────┘
                    │
                    ▼                    S3
┌─────────────────────────────────────────┐
│        PERFORM FREQUENCY UPRATING        │
│      ON AMPLITUDE TEMPORAL CHANGE        │
│          OR PHASE TEMPORAL CHANGE        │
└─────────────────────────────────────────┘
                    │
                    ▼                    S4
┌─────────────────────────────────────────┐
│       PERFORM AMPLITUDE MODULATION       │
│          OR FREQUENCY MODULATION         │
│           ON SOUND SIGNAL BASED ON       │
│        AMPLITUDE TEMPORAL CHANGE         │
│          OR PHASE TEMPORAL CHANGE        │
│            AFTER FREQUENCY UPRATING      │
└─────────────────────────────────────────┘
                    │
                    ▼                    S5
┌─────────────────────────────────────────┐
│            OUTPUT SOUND SIGNAL           │
│        AFTER AMPLITUDE MODULATION        │
│          OR FREQUENCY MODULATION         │
└─────────────────────────────────────────┘
                    │
                    ▼
┌─────────────────────────────────────────┐
│       END HEARTBEAT/RESPIRATION OUTPUT   │
└─────────────────────────────────────────┘
```

FIG. 2

FIG. 3

[4]

RESPI-RATION

TIME [sec]

50

40

30

20

10

0

S

B

$-$ ⟵ 0 ⟶ $+$

FREQUENCY [Hz]

0

FREQUENCY [Hz]

1

(ci[n], cq[n])

$b_r[n]$

TEMPORAL CHANGE CALCULATION UNIT 2

$\sqrt{ci[n]^2 + cq[n]^2}$

$\tan^{-1}(cq[n]/ci[n])$

$a_r[n]$

$\theta_r[n]$

FIG. 4

FIG. 5

[6]

| HEARTBEAT OUTPUT (SIMULTANEOUS RESPIRATION OUTPUT IS POSSIBLE) | MODULATED INFORMATION | AMPLITUDE TEMPORAL CHANGE: $\sqrt{li[n]^2 + lq[n]^2}$, $\sqrt{ri[n]^2 + rq[n]^2}$, $a_p[n]$ |
|---|---|---|
| | | PHASE TEMPORAL CHANGE: $\tan^{-1}(lq[n]/li[n])$, $\tan^{-1}(rq[n]/ri[n])$, $\theta_p[n]$, $2(\theta_p[n]+\phi)+\pi$ |
| | MODULATION METHOD | AMPLITUDE MODULATION OR FREQUENCY MODULATION (DIFFERENT MODULATION IS POSSIBLE FOR HEARTBEAT OUTPUT AND RESPIRATION OUTPUT) |
| RESPIRATION OUTPUT (SIMULTANEOUS HEARTBEAT OUTPUT IS POSSIBLE) | MODULATED INFORMATION | AMPLITUDE TEMPORAL CHANGE: $\sqrt{ci[n]^2 + cq[n]^2}$, $a_r[n]$ |
| | | PHASE TEMPORAL CHANGE: $\tan^{-1}(cq[n]/ci[n])$, $\theta_r[n]$, $2\theta_r[n]-\pi$ |
| | MODULATION METHOD | AMPLITUDE MODULATION OR FREQUENCY MODULATION (DIFFERENT MODULATION IS POSSIBLE FOR RESPIRATION OUTPUT AND HEARTBEAT OUTPUT) |

FIG. 6

EP 4 413 918 A1

FIG. 7

SAMPLING FREQUENCY
< AUDIBLE BAND FREQUENCY

AMPLITUDE OR PHASE

TIME

⇩ COPY INTERPOLATION

UPRATING FREQUENCY
> AUDIBLE BAND FREQUENCY

AMPLITUDE OR PHASE

TIME

EP 4 413 918 A1

FIG. 8

SAMPLING FREQUENCY
< AUDIBLE BAND FREQUENCY

AMPLITUDE OR PHASE

TIME

SPLINE INTERPOLATION

UPRATING FREQUENCY
> AUDIBLE BAND FREQUENCY

AMPLITUDE OR PHASE

TIME

EP 4 413 918 A1

FIG. 9

FIG. 10

[10]

AMPLITUDE
OR PHASE
(AFTER UPDATING)

SOUND SIGNAL
(AFTER AMPLITUDE
MODULATION)

SOUND SIGNAL
(AFTER FREQUENCY
MODULATION)

TIME

HEARTBEAT AMPLITUDE (AFTER UPRATING)

TIME

RESPIRATION PHASE (AFTER UPRATING)

TIME

SOUND SIGNAL (AFTER MODULATION)

AMPLITUDE MODULATION BY HEARTBEAT AMPLITUDE

TIME

FREQUENCY MODULATION BY RESPIRATION PHASE

FIG. 11

**EP 4 413 918 A1**

## INTERNATIONAL SEARCH REPORT

| International application No. |
|---|
| **PCT/JP2021/036642** |

**A. CLASSIFICATION OF SUBJECT MATTER**

*A61B 5/0245*(2006.01)i; *A61B 5/11*(2006.01)i
FI: A61B5/11 110; A61B5/0245 Z

According to International Patent Classification (IPC) or to both national classification and IPC

**B. FIELDS SEARCHED**

Minimum documentation searched (classification system followed by classification symbols)

A61B5/0245; A61B5/11

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| Y | JP 2009-538720 A (BIANCAMED LTD) 12 November 2009 (2009-11-12) paragraphs [0017]-[0103] | 1–6 |
| Y | JP 2019-146772 A (UNIV FUKUI) 05 September 2019 (2019-09-05) paragraph [0002] | 1–6 |
| Y | JP 2012-524627 A (LIFEWAVE INC) 18 October 2012 (2012-10-18) paragraphs [0050], [0054], [0099], [0100] | 1–6 |
| Y | US 2002/0120184 A1 (BECK, Eric C.) 29 August 2002 (2002-08-29) paragraphs [0050]-[0053] | 1–6 |
| Y | JP 2013-538602 A (KONINKL PHILIPS ELECTRONICS NV) 17 October 2013 (2013-10-17) paragraph [0043] | 2-6 |
| Y | JP 2013-172899 A (TOYOTA INFOTECHNOLOGY CENTER CO LTD) 05 September 2013 (2013-09-05) paragraph [0045] | 3-6 |

☑ Further documents are listed in the continuation of Box C.   ☑ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **13 December 2021** | **21 December 2021** |

| Name and mailing address of the ISA/JP | Authorized officer |
|---|---|
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

**INTERNATIONAL SEARCH REPORT**

| International application No. |
| --- |
| **PCT/JP2021/036642** |

**C.      DOCUMENTS CONSIDERED TO BE RELEVANT**

| Category* | Citation of document, with indication, where appropriate, of the relevant passages | Relevant to claim No. |
| --- | --- | --- |
| A | JP 2012-239748 A (TOYOTA CENTRAL R&D LABS INC) 10 December 2012 (2012-12-10) paragraphs [0074]-[0090] | 1–6 |

Form PCT/ISA/210 (second sheet) (January 2015)

## INTERNATIONAL SEARCH REPORT
### Information on patent family members

| | International application No. |
|---|---|
| | **PCT/JP2021/036642** |

| Patent document cited in search report | | | Publication date (day/month/year) | Patent family member(s) | | | Publication date (day/month/year) |
|---|---|---|---|---|---|---|---|
| JP | 2009-538720 | A | 12 November 2009 | US 2009/0203972 A1 paragraphs [0017]-[0116] WO 2007/143535 A2 EP 3616611 A1 CA 2654095 A1 CN 101489478 A | | | |
| JP | 2019-146772 | A | 05 September 2019 | (Family: none) | | | |
| JP | 2012-524627 | A | 18 October 2012 | US 2010/0274145 A1 paragraphs [0061], [0065], [0108]-[0109] WO 2010/124117 A2 CN 102421371 A | | | |
| US | 2002/0120184 | A1 | 29 August 2002 | (Family: none) | | | |
| JP | 2013-538602 | A | 17 October 2013 | US 2013/0135137 A1 paragraph [0055] WO 2012/020365 A1 EP 2417908 A1 CN 103068304 A | | | |
| JP | 2013-172899 | A | 05 September 2013 | (Family: none) | | | |
| JP | 2012-239748 | A | 10 December 2012 | (Family: none) | | | |

Form PCT/ISA/210 (patent family annex) (January 2015)

**EP 4 413 918 A1**

**Patent documents cited in the description**

- JP 2019129996 A